# EUROPEAN PATENT SPECIFICATION

(11) **EP 2 222 284 B1**
(45) Date of publication and mention of the grant of the patent: **01.07.2020**
(21) Application number: 08852557.1
(22) Date of filing: 18.11.2008
(51) Int. Cl.: A61K 9/58

(54) **PROLONGED RELEASE OF LOCAL ANESTHETICS USING MICROPARTICLES AND SURGERY APPLICATIONS**
VERLÄNGERTE FREISETZUNG VON LOKALANÄSTHETIKA MIT MIKROPARTIKELN UND OPERATIVE ANWENDUNGEN
LIBÉRATION PROLONGÉE D'ANESTHÉSIQUES LOCAUX AU MOYEN DE MICROPARTICULES ET APPLICATIONS CHIRURGICALES

(30) Priority: 19.11.2007 US 989098 P
(43) Date of publication of application: 01.09.2010
(73) Proprietor: Capsulated Systems Inc., Miamisburg, OH 45342-4449 (US)
(72) Inventor: LYNCH, Roland, M., Ithaca NY 14850 (US); MASINDE, Lwandiko, E., Hampton VA 23669 (US)
(74) Representative: Dauncey, Mark Peter
(86) International application number: PCT/US2008/083940
(87) International publication number: WO 2009/067462

(56) References cited:
- WO-A1-94/05265
- WO-A2-2009/129509
- US-A- 5 654 008
- US-A1- 2003 152 637
- US-B1- 6 248 345

## Description

### BACKGROUND

Microparticle encapsulation is an important technology that can provide a mechanism to deliver pharmaceutical agents *in vivo.* Microparticles can be made from a variety of biological and synthetic materials, and can have a wide range of properties. Microparticles can also be made by numerous methods, including solvent evaporation, and can be placed in aqueous suspensions. See for example Masinde et al., International Journal of Pharmaceutics (1993), 100:121-131. Moreover, microparticles can encapsulate a variety of pharmaceutical agents.

Microparticle encapsulation can be used to deliver drugs to treat a variety of biological symptoms. For example, U.S. Patent Nos. 6,426,339; 5,618,563; and 5,47,060, describe microparticle encapsulation for treating different types of conditions. One type of condition is pain management and, in particular, pain management post-surgery (postoperative analgesia). In many cases, injection of local anesthetic is needed.

Sustained and controlled release is an important aspect of drug delivery. See for example Ed. J. R. Robinson (1978) Sustained and Controlled Release Drug Delivery Systems, including chapter 5 on "Pathological Evaluation of Injection Injury", pages 351-410.

Pain management after surgery often starts with an injection of a local anesthetic as part of surgery. This, however, provides pain relief for only a matter of hours after surgery for a single injection, even for local anesthetics which are deemed relatively longer lasting. See for example US Patent No. 5,618,563. In many cases, an augmentation agent is believed needed to extend the action of the local anesthetic. See for example US Patent Nos. 5,618,563 and 5,747,060. The patient can then be prescribed medications such as hydrocodone, percoset, vicadin, or other opiates or opiate-like materials. Opiates operate on the central nervous system to manage pain for the next 5-7 days, after which the pain subsides to a level that can be controlled by over-the-counter pain killers such as ibuprophen, acetaminophen, or aspirin. However, opiates present potential problems with addiction, abuse, adverse reaction, and limiting of patient activity.

WO2009/129509A2 describes an implantable drug depot useful for reducing, preventing or treating post-operative pain in a patient in need of such treatment, the implantable drug depot comprising a polymer and a therapeutically effective amount of a local anesthetic or pharmaceutically acceptable salt thereof, wherein the drug depot is implantable at a site beneath the skin to reduce, prevent or treat post-operative pain, and the drug depot is capable of releasing (i) a bolus dose of the local anesthetic or pharmaceutically acceptable salt thereof at a site beneath the skin and (ii) a sustained release dose of an effective amount of the local anesthetic or pharmaceutically acceptable salt thereof over a period of at least 4 days.

US2003/0152637A1 describes pharmaceutical formulations administered *via* parenteral methods, which provide a prolonged localized analgesic effect, in particular, formulations comprising a pharmaceutically acceptable biocompatible biodegradable carrier containing a local anesthetic and the parenteral administration of such carrier in a manner such that a localized analgesic effect is attained for a prolonged period of time.

US6248345 describes sustained release local anesthetic formulations that are administered intra articularly and/or into body spaces/cavities. The formulation is preferably a plurality of injectable microparticles including a local anesthetic and an effective amount of a biocompatible, biodegradable, sustained release material prolonging the release of the local anesthetic and optionally and a pharmaceutically acceptable, i.e., non-toxic, augmenting agent effective to prolong the duration of the local anesthesia for a time period longer than that obtainable without the augmenting agent.

WO94/05265 describes an improved biodegradable controlled release system consisting of a polymeric matrix incorporating a local anesthetic for the prolonged administration of the local anesthetic agent, and a method for the manufacture thereof, are disclosed. The polymers and method of manufacture used to form the matrices are selected on the basis of their degradation profiles: release of the topical anesthetic in a linear, controlled manner over a period of preferably two weeks and degradation (in vivo) with a half-life of less than six months, more preferably two weeks, to avoid localized inflammation. Alternatively, a non-inflammatory can be incorporated into the polymer with the local anesthetic to prevent inflammation.

US5654008 describes a process for preparing biodegradable microparticles comprising a biodegradable polymeric binder and a biologically active agent. A first phase, comprising the active agent and the polymer, and a second phase are pumped through a static mixer into a quench liquid to form microparticles containing the active agent. Preferably, a blend of at least two substantially non-toxic solvents, free of halogenated hydrocarbons, is used to dissolve or disperse the agent and dissolve the polymer.

Long-term local pain relief may be indicated for a wide variety of conditions in humans, including but not limited to: open reduction of fractures with internal fixation; reductions of fractures generally; injection of therapeutic substances into joints or ligaments; removal of implanted devices from bone; bunionectomy; treatment of toe deformities generally; knee arthroscopy; arthroscopy generally; division of joint capsule ligament, or cartilage; excision of semilunar cartilage of knee; synovectomy; other incision and excision of joint structure; total hip replacement; total knee replacement; repair of knee generally; repair of joints generally; excision of lesion of muscle, tendon, fascia, and bursa; other operations generally on muscles, tendons, fascia, and bursa; amputation of upper limb; amputation of lower limb; and other operations generally on the musculoskeletal system.

Long-term local pain relief may also be warranted in the preemptive management of chronic pain associated with a variety of conditions in humans, including but not limited to: burns, cancer, epidural, femoral breaks, reflex sympathetic dystrophy, and complex regional pain syndrome.

Long-term local pain relief may also be indicated for a variety of conditions in animals, including but not limited to: anterior cruciate ligament (ACL) surgery, cranial cruciate ligament (CCL) surgery; hip replacements, knee replacements; trauma to extremities; burns; and declawing.

A need exists to find better, more efficient pain management approaches, including longer lasting pain relief from local anesthetics which can eliminate or reduce the need for opiate usage and reduce or eliminate side effects. This is particularly true when there are limits on the volume of anesthetic which can be injected. Furthermore, a need exists for prolonged local anesthetics that do not require augmentation agents. Some augmentation agents are condition-specific for particular deceases, such as cancer, or others, such as steroids, are prone to produce side effects.

### SUMMARY

Methods of making, methods of using, and compositions are described herein for producing an extended and controlled drug release profile. According to a first aspect of the present invention, there is provided a composition comprising: a plurality of microparticles, wherein substantially all of the plurality of microparticles comprise one or more local anesthetic compounds, wherein at least some of the plurality of microparticles comprise at least one polymer from which the local anesthetic compound is releasable, wherein at least some of the plurality of microparticles comprise one or more local anesthetic compounds in an amount of at least 70 % by weight, wherein the average amount of local anesthetic compound in the composition is at least 50 % by weight, and wherein the composition optionally further comprises a local anesthetic effect-augmentation agent which, if present, is included in the composition in an amount of less than 0.005 % by weight, characterized in that the plurality of microparticles comprises a mixture of at least two groups of microparticles, each group having an average polymer molecular weight, an average drug loading percentage, and an average particle size, wherein at least the average polymer molecular weight is different in each of the groups, wherein at least one of the local anesthetic compounds is chosen from the group consisting of:
lidocaine, bupivacaine, ropivacaine, dibucaine, etidocaine, tetracaine, xylocaine, procaine, chloroprocaine, prilocaine, mepivacaine, mixtures thereof and salts thereof, and wherein the at least one polymer is chosen from the group consisting of polyesters, polyorthoesters, proteins, polysaccharides, and combinations thereof, poly(lactic) acid, poly(glycolic) acid, polyactide,polyglycolides, poly(DL-lactic-co-glycolic) acid, polyanhydride, polycaprolactone, and polyphosphazene.

Another embodiment provides a composition comprising: (a) a first group of microparticles, each microparticle in said first group having a molecular weight greater than 91,600 Daltons, a particle size between 20 and 50 microns, and a drug loading of at least one anesthetic of 80% by weight; (b) a second group of microparticles, each microparticle in said second group having a molecular weight between 57,600 and 91,600 Daltons, a particle size between 70 and 100 microns, and a drug loading of said at least one anesthetic of 75% by weight; (c) a third group of microparticles, each microparticle in said third group having a molecular weight between 31,300 and 57,600 Daltons a particle size between 100 and 120 microns, and a drug loading of said at least one anesthetic of 50% by weight; and (d) a fourth group of microparticles, each microparticle in said fourth group having a molecular weight between 5,000 and 12,900 Daltons, a particle size greater than 120 microns, and a drug loading of said at least one anesthetic of 30% by weight, wherein said first group comprises 30%, said second group comprises 40%, said third group comprises 20%, and said fourth group comprises 10% of the total microparticles of all four groups.

Another embodiment provides a composition comprising: (a) a first group of microparticles, each microparticle in said first group having a molecular weight between 57,600 and 91,600 Daltons, a particle size between 70 and 100 microns, and a drug loading of at least one anesthetic of 80% by weight; (b) a second group of microparticles, each microparticle in said second group having a molecular weight between 57,600 and 91,600 Daltons, a particle size between 70 and 100 microns, and a drug loading of said at least one anesthetic of 80% by weight; and (c) said at least one anesthetic in free form, each anesthetic particle in free form having a particle size between 50 and 100 microns, wherein said first group comprises 47%, said second group comprises 47%, and the free form anesthetic comprising 6% of the total mass of elements (a), (b), and (c).

Another embodiment provides a composition comprising: (a) a first group of microparticles, each microparticle in said first group having a molecular weight greater than 91,600 Daltons, a particle size between 20 and 50 microns, and a drug loading of at least one anesthetic of 58% by weight; (b) a second group of microparticles, each microparticle in said second group having a molecular weight between 57,600 and 91,600 Daltons, a particle size between 70 and 100 microns, and a drug loading of said at least one anesthetic of 80% by weight; (c) a third group of microparticles, each microparticle in said third group having a molecular weight between 5,000 and 12,900 Daltons, a particle size between 100 and 120 microns, and a drug loading of said at least one anesthetic of 70% by weight; and (d) a fourth group of microparticles, each microparticle in said fourth group having a molecular weight between 5,000 and 12,900 Daltons, a particle size between 100 and 120 microns, and a drug loading of said at least one anesthetic of about 70% by weight, wherein said first group comprises 20%, said second group comprises 20%, said third group comprises 40%, and said fourth group comprises 20% of the total microparticles of all four groups.

In an embodiment, the composition is totally free of local anesthetic effect-augmentation agents.

In an embodiment, at least some of the plurality of microparticles comprises the local anesthetic compound substantially free of the polymer.

In an embodiment, substantially all of the microparticles that comprise polymer also comprise at least 70% by weight of the local anesthetic compound.

In an embodiment, at least 90% by weight of the microparticles comprise at least 60% by weight of the local anesthetic compound.

In an embodiment, the at least one polymer is poly(DL-lactic-co-glycolic) acid.

In an embodiment, at least one of the local anesthetic compounds is lidocaine.

In an embodiment, the at least one polymer is poly(DL-lactic-co-glycolic) acid and at least one of the local anesthetic compounds is lidocaine.

According to a second aspect of the present invention, there is provided a method of making drug loaded microparticles, comprising: (a) providing at least one anesthetic chosen from the group consisting of: lidocaine, bupivacaine, ropivacaine, dibucaine, etidocaine, tetracaine, xylocaine, procaine, chloroprocaine, prilocaine, mepivacaine, mixtures thereof and salts thereof;; (b) providing at least one polymer chosen from the group consisting of polyesters, polyorthoesters, proteins, polysaccharides, and combinations thereof, poly(lactic) acid, poly(glycolic) acid, polyactide, polyglycolide, poly(DL-lactic-co-glycolic) acid, polyanhydride, polycaprolactone, and polyphosphazene; (c) dissolving said at least one anesthetic and said at least one polymer in an organic solvent to produce a solution; (d) emulsifying said solution by stirring it into an aqueous medium to form an oil-in-water emulsion; (e) evaporating said organic solvent to allow said at least one anesthetic and said at least one polymer to harden into microparticles; and (f) repeating steps (a) through (e) to produce multiple batches of microparticles, wherein each batch comprises microparticles within a distinct size range, wherein each batch makes up a different percentage of the combination of all of the batches, and wherein each batch comprises said at least one anesthetic at a different loading level.

Also described is a method of using drug loaded microparticles, comprising: (a) providing a solution comprising multiple batches of microparticles loaded with at least one anesthetic and (b) injecting said microparticles into a body cavity, wherein each batch comprises microparticles within a distinct size range, wherein each batch makes up a different percentage of the combination of all of the batches, and wherein each batch comprises said at least one anesthetic at a different loading level.

Also described is a method of using drug loaded microparticles, comprising: (a) providing a powder comprising multiple batches of microparticles loaded with at least one anesthetic and (b) depositing said microparticles into a body cavity, wherein each batch comprises microparticles within a distinct size range, wherein each batch makes up a different percentage of the combination of all of the batches, and wherein each batch comprises said at least one anesthetic at a different loading level.

Also described is a method of providing pain relief in the recovery from surgery, said method comprising: (a) providing a solution comprising multiple batches of microparticles loaded with at least one anesthetic and (b) injecting said microparticles into a body cavity, wherein each batch comprises microparticles within a distinct size range, wherein each batch makes up a different percentage of the combination of all of the batches, and wherein each batch comprises said at least one anesthetic at a different loading level.

Also described is a method of providing pain relief in the recovery from surgery, said method comprising: (a) providing a powder comprising multiple batches of microparticles loaded with at least one anesthetic and (b) depositing said microparticles into a body cavity, wherein each batch comprises microparticles within a distinct size range, wherein each batch makes up a different percentage of the combination of all of the batches, and wherein each batch comprises said at least one anesthetic at a different loading level.

Also described is a method of providing chronic pain relief, said method comprising: (a) providing a solution comprising multiple batches of microparticles loaded with at least one anesthetic and (b) injecting said microparticles into a body cavity, wherein each batch comprises microparticles within a distinct size range, wherein each batch makes up a different percentage of the combination of all of the batches, and wherein each batch comprises said at least one anesthetic at a different loading level.

Also described is a method of providing chronic pain relief, said method comprising: (a) providing a powder comprising multiple batches of microparticles loaded with at least one anesthetic and (b) depositing said microparticles into a body cavity, wherein each batch comprises microparticles within a distinct size range, wherein each batch makes up a different percentage of the combination of all of the batches, and wherein each batch comprises said at least one anesthetic at a different loading level.

One or more embodiments described herein can provide one or more of the following advantages.

For example, one possible advantage is extended relief from pain.

Another possible advantage is the ability to reduce or eliminate the need for augmentation agents, epinephrine and other vasoconstrictors.

Another possible advantage is that the microparticles can be lidocaine-based.

Another possible advantage is that the microparticles are injectable through an 18 gauge needle.

Another possible advantage is that the microparticles can provide continuous pain relief for at least 6 days post-surgery.

Another possible advantage is that the microparticles allow for full sensory response recovery.

Another possible advantage is that the microparticles cause no nerve nor tissue damage.

Another possible advantage is that the microparticles cause minimal motor response suppression.

Another possible advantage is that the polymer is quickly and fully absorbable in a few days time period, and not in terms of months.

Another possible advantage is that the microparticles do not cause side effects.

Another possible advantage is that the microparticles minimize the need for opiates and opiate-like medications.

Another possible advantage is that the microparticles supersede side effects of opiates.

Another possible advantage is that the microparticles supersede the potential for misuse and abuse of opiates.

Another possible advantage is that the microparticles allow for speedy recovery and physical therapy post-surgery.

Another possible advantage is that all the components of the microparticles are FDA approved.

### BRIEF DESCRIPTION OF THE DRAWINGS

Figure 1 illustrates the *in vitro* release of free lidocaine (lidocaine free base).
Figure 2 illustrates the *in vitro* release of lidocaine from low molecular weight Poly(DL-lactic-co-glycolic acid) (DL-PLG) (D1) microparticles with 80% lidocaine loading.
Figure 3 illustrates the *in vitro* release of lidocaine from medium molecular weight DL-PLG microparticles (D3) with 80% loading.
Figure 4 illustrates the *in vitro* release of lidocaine from high molecular weight DL-PLG microparticles (D4) with 80% loading.
Figure 5 illustrates the *in vitro* release of lidocaine from high molecular weight DL-PLG microparticles (D5) with 80% loading.
Figure 6 illustrates the *in vitro* release of lidocaine from a combination of four different DL-PLG microparticles in accordance with the present invention.
Figure 7 illustrates the *in vitro* release of lidocaine from a combination of two batches of the same DL-PLG microparticles and free lidocaine in accordance with the present invention.
Figure 8 illustrates the *in vitro* release of lidocaine from a combination of two batches of the same DL-PLG microparticles and free lidocaine in accordance with the present invention.
Figure 9 illustrates the *in vitro* release of lidocaine from a combination of three different DL-PLG microparticles, with two batches of one of the microparticles (D1) in accordance with the present invention.
Figure 10 illustrates electron microscope pictures of D1 microparticles loaded with 80% lidocaine in accordance with the present invention.
Figure 11 illustrates electron microscope pictures of D2 microparticles loaded with 80% lidocaine in accordance with the present invention.
Figure 12 illustrates electron microscope pictures of D3 microparticles loaded with 80% lidocaine in accordance with the present invention.
Figure 13 illustrates electron microscope pictures of D4 microparticles loaded with 80% lidocaine in accordance with the present invention.

### DETAILED DESCRIPTION

### INTRODUCTION

Provided herein includes a method to deliver a mixture of high local anesthetic loaded microparticles (70-80% by weight) to obtain maximum pain relief by providing an extended release curve to get patients past the 3-day window where they would normally need an opiate. By providing a combination of microparticles that releases anesthetics at different times and different rates, an aggregate release profile can be produced. This profile can be tailored to produce a desired temporal delivery of the anesthetic.

Aggregate release profiles can also be produced with combinations of microparticles of different sizes. For microparticles made of polymers, the molecular weight of the polymers has an effect on how drugs encapsulated within the microparticles are released. Generally, low molecular weight polymers release drugs earlier than high molecular weight polymers. The diffusion rate of drugs, i.e. lidocaine, through the polymer is constant. By combining microparticles with different molecular weights to provide an overlap of early and late drug release, an aggregated, extended drug release can be produced.

Also, for the situation in which glycolic acid and lactic acid are used as monomers for creating polymer microparticles, higher ratios of glycolic acid to lactic acid in the polymer lead to a shorter degradation period of the polymer (because glycolic acid is more brittle than lactic acid). This trend therefore causes the polymer to break down faster after drug release. For example, a 50:50 poly(DL-lactic-co-glycolic) acid (DL-PLG) microparticle, i.e. 50% lactic acid and 50% glycolic acid, will degrade faster than a 75:25 DL-PLG, i.e. a 75% lactic acid and 25% glycolic acid.

In addition to determining the combinations necessary to produce extended drug release, provided herein includes a method to obtain high loading levels. In order to provide extended drug release past the point where normal drug injections wear off, at least some of the microparticles should be loaded at high drug levels, including a drug loading of up to 80%. This loading was produced keeping in mind the limitations that are presented with drug injections. Drug injections *in vivo* are limited by the space available in the body space of the injection site to accommodate such injections. Typically, 5-10 ml of liquid volume is the standard amount that can be injected in the great majority of body spaces, although some spaces can tolerate up to 25-30 ml. Therefore, in order to inject microparticles in a liquid volume within the range of 5-10 ml, there should be a balance between particle mass and drug loading. If too much weight of microparticles are suspended in the liquid volume, then the suspension may not be injectable. However, if too few microparticles are suspended, then the drug dose will not be high enough to produce an effect and the requisite duration of release. If the molecular weight of the polymer is too low, at higher drug loading, the microparticles will be tacky and form fused masses that can not be injected. In recognizing this balance, a method was produced to obtain maximum drug loading up to 80% while reducing the total powder in a liquid volume suitable for injection.

### MICROPARTICLES

Microparticles are known in the art. Microparticles include any particle capable of encapsulating and releasing drugs, including pellets, rods, pastes, slabs, spheres, capsules, beads, microparticles, microcapsules, microbeads, nanocapsules, and nanospheres.

Microparticles can also be formed into any shape. In one embodiment, the shape is spherical, oval, or elliptical. In another embodiment, the shape is random.

Microparticles can be made from a variety of materials, including synthetic and natural materials. The microparticles are made from at least one polymer.

### POLYMERS

Polymers including synthetic polymers are known in the art. Polymers capable of being formed into microparticles include homopolymers and copolymers. Examples of homopolymers include poly(lactic) acid and poly(glycolic) acid. Other classes of polymers applicable to the invention include but are not limited to polyesters, polyorthoesters, proteins, polysaccharides, and combinations thereof. In one embodiment, the polymers can be prepared from the polymers disclosed in U.S. Patent No. 5,922,340, , including polylactide, polyglycolide, poly(DL-lactic-co-glycolic) acid, polyanhydride, polyorthoester, polycaprolactone, and polyphosphazene.

### LOCAL ANESTHETIC COMPOUNDS

In the present invention, a local anesthetic is provided with the microparticles.

Anesthetics may be selected from lidocaine, bupivacaine, ropivacaine, dibucaine, etidocaine, tetracaine, xylocaine, procaine, chloroprocaine, prilocaine, mepivacaine, mixtures thereof, and salts thereof.

### AUGMENTATION AGENTS

Augmentation agents include agents that prolong the effect of local anesthetic compounds. Augmentation agents include glucocorticosteroids, alphaxalone, allotetrahydrocortisone, aminopyrine, benzamil, clonidine, minoxidil, dehydroepiandrosterone, dextran, diazepam, diazoxide, ouabain, digoxin, spantide, taxol, tetracthylammonimu, valproic acid, vincritine, and active derivatives, analogs, and mixtures thereof, as indicated in U.S. Patent Nos. 6,451,335 and 6,534,081.

In one embodiment, augmentation agent is not used.

### SUBSTANTIALLY FREE

The compositions are substantially free of augmentation agents. For example, compositions which are substantially free include those where augmentation agent is present less than about 0.005%, as described in U.S. Patent No. 5,922,340.

### MAKING MICROPARTICLES AND MICROPARTICLES LOADED WITH DRUGS

Microparticles can be prepared using the solvent evaporation method or any other suitable method such as hot melt. In the solvent evaporation method, local anesthetic and polymer can be dissolved in a common organic solvent to produce a solution. This solution can then be emulsified by stirring it into an aqueous medium containing an emulsifying agent to form an oil-in-water emulsion. The organic solvent can then be evaporated, causing the remaining anesthetic and polymer to harden into microparticles.

In one embodiment, a compact solid microparticle with smooth surfaces is provided.

In another embodiment, application of vacuum to the emulsion during the evaporation stage produces pores in the microparticle. The pores can be on the surface and within the microparticle interior.

In another embodiment, the microparticle size is altered by applying different stirring rates during the emulsification process.

In another embodiment, the microparticle size, including diameter, ranges from about 20 to about 150 microns.

In another embodiment, the anesthetic is loaded at different levels in the range from about 20 to about 80 percent.

The microparticles have different molecular weights.

In another embodiment, the microparticle has a molecular weight range from about 5,000 to about 122,000 Daltons.

In another embodiment, the microparticle is made of a co-polymer. An example of a co-polymer is poly(DL-lactic-co-glycolic) acid (DL-PLG).

In another embodiment, the co-polymer microparticle has ratios between 25:75 and 75:25.

In another embodiment, the microparticle is suspended in a pharmaceutically acceptable medium for injection.

In another embodiment, the microparticle is a dry powder and is deposited in a body space.

Microparticles loaded with drugs can be prepared by dissolving polymers and drugs in a first solvent. The first solvent can be mixed with a second solvent and the resulting mixture shaken. The mixture can then be transferred into a further solution containing the second solvent and stirred to allow evaporation of the first solvent. Suspended microparticles can then be allowed to sediment, the resulting supernatant decanted, and the microparticles collected by centrifuging.

### MICROPARTICLE COMBINATIONS

A combination of different types of microparticles is provided. The combination includes different blends, or mixtures, of microparticles and drugs.

In another embodiment, the combination includes a mixture of microparticles made of the same material. For example, microparticles can all be poly(lactic)acid or poly(glycolic) acid.

In another embodiment, the combination includes a mixture of microparticles having different materials. For example, microparticles can be different molecular weights ofpoly(DL-lactic-go-glycolic) acid (DL-PLG).

In another embodiment, the combination includes a mixture of microparticles with different diameters and/or with different loading levels of drugs.

In another embodiment, the mixture of microparticles comprises classes of microparticles that comprise a different percentage of the entire mixture. For example, a mixture can include 30% of purely poly(lactic)acid microparticles and 70% of purely poly(glycolic)acid.

In another embodiment, the combination includes microparticles mixed with free drugs.

In another embodiment, the mixture of microparticles comprises classes of microparticles made of differing molecular weights

In another embodiment, the mixture of microparticles comprises classes of microparticles made of differing loading percentages

### INJECTABLE FORMULATIONS

The microparticle combinations can be provided in a suspension with a pharmaceutically acceptable medium. The microparticles can be administered into a body space, including the pleura, peritoneum, cranium, mediastinum, peridcardium, bursae, epidural space, intrathecal space, and intraocular space or deposited proximal to a nerve fiber or nerve trunks.

In one embodiment, the microparticle combination is injected at or near selected nerves.

In another embodiment, the microparticle combination is injected within 1-2 mm of peroneal, tibial or sciatic nerves using a locator needle.

In another embodiment, the microparticle combination is kept in a refrigerator until mixed in a suspension of the pharmaceutically acceptable medium.

In another embodiment, the microparticle combination is delivered as dry powder without a medium.

In another embodiment, the microparticle combination does not include an augmenting agent.

In another embodiment, the microparticle combination is injected only once.

Other embodiments are illustrated in the following examples.

### APPLICATIONS/SURGERIES

The compositions can be used in surgeries including surgeries for which long term local anesthetics are indicated for.

### Human Orthopedic Surgery of Extremities

Open Reduction of fracture with internal fixation
Other reduction of fracture
Injection of therapeutic substance into joints or ligament
Removal of implanted devices from bone
Bunionectomy
Other toe deformities
Arthroscopy of knee
Other arthroscopy
Division of joint capsule, ligament, or cartilage
Excision of semilunar cartilage of knee
Synovectomy
Other incision and excision of joint structure
Total Hip Replacement
Total Knee Replacement
Other Repair of Knee
Other repair of joints
Excision of lesion of muscle, tendon, fascia & bursa
Other operations/muscles, tendons, fascia and bursa
Ampution of upper limb
Amputation of lower limb
Other operations on the musculoskeletal system

Examples of human premptive chronic pain management include, for example, burns, cancer, epidural, femoral breaks, and RSD(Reflex Sympathetic Dystrophy or Complex Regional Pain Syndrome).

Examples of companion animal surgeries include, for example, ACL/CCL surgeries, hip replacements, knee replacements, trauma to extremities, burns, and cat de-clawments.

### EXAMPLE 1: IN VITRO TEST METHODS

Microparticle batches in an amount of 100 mg were placed in a dialysis tube (high retention seamless cellulose tubing; 23 mm x 15 mm, MW cut-off 05173; Sigma Aldrich). The tube was then placed in a 30 ml glass vial containing 10 ml of deionized ultra-filtered water (Fisher Scientific). Vials were placed in a reciprocating shaking bath (Reciprocating Shaking Bath Model 50; Precision Scientific) with the temperature adjusted to 37 °C, and shaking speed of 100 rpm.

Samples for drug release analysis were drawn at time intervals of 0, 0.5, 2, 4, and 12 hours and continued as shown in the drug release profiles of Figures 1-5. The entire 10 ml of dissolution medium was replaced with fresh medium at each sampling time interval. Dilution of 0.1 ml of the withdrawn sample was diluted to 10 ml of water in clean culture tubes of borosilicate glass (Pyrex). The sample was measured for drug content by UV absorbance at 214 nm using a UV-spectrophotometer (Lambda 3 spectrophotometer Model R100A; Perking Elmer). Two samples per microparticle batch were measured for drug release and triplicate samples were prepared for each release interval for UV-absorbance.

### EXAMPLE 2: IN VIVO INJECTION AND TEST METHODS

*In vivo* tests were performed to compare the duration of pain relief between microparticle preparations and conventional lidocaine. Using doses determined in a previous pilot study (data not shown), 6 sheep underwent a blinded, randomized crossover study using a closed envelop technique. The sheep were injected at two time points, one time point with microparticle preparations and the other with conventional lidocaine. The order in which the microparticle preparations and conventional lidocaine were injected were randomized. The first injection was made near the common peroneal nerve on one hind leg. The interval between injections were at least 2 weeks, giving enough time for all signs of drug action from the first injection to disappear before the second injection was made into the contralateral nerve, i.e. peroneal nerve of the opposite hind leg. In order to describe the pharmacokinetics of each group, serial jugular blood samples of 2 ml each were collected. Observations were made of motor and sensory block, or a lack thereof, at durations of 15, 30, and 45 minutes, and at 1, 2, 4, 8, 12, 16, 20, and 24 hours. After this, observations were made at 12 hour intervals. Analgesia was measured by clamping the skin of the cranial aspect, proximal to every toe (common peroneal dermatomes).

The perineural injection used in all of these experiments was performed under general anesthesia to assure minimal discomfort to the sheep during the step of locating the nerve, and to assure maximum accuracy for depositing local anesthetic. The entire procedure was performed under sterile conditions, i.e. skin clipped and washed at least three times with chlorhexidine soap, hands in sterile gloves, and perimeter barrier with sterile drapes. The nerve was located using electrolocation, a standard procedure used on patients in which an insulated needle (18 gauge) with a small, electrically conductive tip was advanced incrementally toward the nerve until movement of the appropriate muscle groups, i.e. flexion of the claws, peroneal response, caused by direct nerve stimulation was elicited with a small current of 0.3 mA. The stimulation current was applied in a square wave at a frequency of 2Hz, which stimulates motor neurons in preference to nociceptive neurons. Once the nerve was located, the preparation was injected, the needle withdrawn, and the sheep allowed to recover from general anesthesia. This procedure generally required less than 15 minutes of general anesthesia.

For injecting the microparticle preparations, the insulated needle and its tube were primed with 2.5 ml of carboxymethyl cellulose sodium solution prior to locating the nerve. This was done to displace the air in the needle assembly. Once the nerve was located, a syringe containing 1.5 mg of microparticles suspended in carboxymethyl cellulose solution to 5 ml was attached to the open end of the tube and an injection was made. To complete the injection, 2.5 ml of air was pushed through the tube to displace the suspension.

### EXAMPLE 3: AMOUNTS INJECTED IN VIVO

The *in vivo* procedure described above is also illustrated in Example 11, which describes the results of the procedure. In one of the experiments, 3.00 g of D4 microparticles, divided into two 1.5 g syringes, was intended to be injected. However, due to injection difficulty, an estimated 2.0 g of powder total was injected.

In another of the experiments, an estimated amount of 2.5 g D4 microparticles, divided into two syringes with 100 mg lidocaine free base, was suspended in 3-5 ml suitable suspending medium and injected.

Figure 1 shows the in vitro release profile of free lidocaine (lidocaine free base). The release profile shows a peak of 18% release at about one day, but then it rapidly tapers off such that the drug is "exhausted" at time point 11, which corresponds to 3 days. The equivalent of 2% of 2.8 g, i.e. 5.6 mg, would be needed to produce sensory suppression. Since there is only 100 mg of lidocaine powder, the equivalent of 5.6 mg would be 5.6% of 100 mg as a minimum required to be released to work. Lidocaine free base falls below that level at point 10, corresponding to 2.5 days.

### EXAMPLE 4: MATERIALS FOR IN VIVO AND IN VITRO DRUG RELEASE FROM MICROPARTICLES

(a) Poly(DL-lactic-co-glycolic) acid (DL-PLG) (Durect Corp, Lactel Absorbable Polymers) (inherent viscosity below in terms of dL/g in HFIP at 30 °C):
   (i) 50:50 DL-PLG at 7,400 MW, 0.15-0.25 inherent viscosity (D1)
   (ii) 50:50 DL-PLG at 28,500 MW, 0.26-0.54 inherent viscosity (D2)
   (iii) 50:50 DL-PLG at 52,400 MW, 0.55-0.75 inherent viscosity (D3)
   (iv) 50:50 DL-PLG at 81,600 MW, 0.76-0.94 inherent viscosity (D4)
   (v) 50:50 DL-PLG at 122,000 MW, 0.95-1.20 inherent viscosity (D5)
(b) Lidocaine powder at greater than 98% purity (L7757; Sigma-Aldrich)
(c) Poly(vinyl alcohol) at 98-99% purity, hydrolyzed (Sigma Aldrich)
(d) Carboxymethyl cellulose, sodium salt, 90,000 avg. MW (Fisher Scientific)
(e) Methylene Chloride (Dichloroethane) at 99.6% purity, A.C.G. reagent (Sigma Aldrich)

### EXAMPLE 5: IN VITRO DRUG RELEASE FROM D1 MICROPARTICLES HAVING LOW MOLECULAR WEIGHT

A batch of low molecular weight microparticles (D1) having drug loading is provided for comparison purposes against the microparticle combination batches described in the following examples.

Figure 2 shows the in vitro release profile of D1 microparticles, exemplifying microparticles made of low molecular weight polymers. This batch is made up of D1 microparticles having an 80% loading of lidocaine. The release profile shows a peak of 20% release at about one day, but then rapidly tapers off such that the drug is "exhausted" at time point 11, which corresponds to 3 days. At 3 days, although the drug is still being released, because of the high loading of D1 microparticles, they were tacky and not suitable for injection.

### EXAMPLE 6: IN VITRO DRUG RELEASE FROM D4 MICROPARTICLES HAVING HIGH MOLECULAR WEIGHT

A batch of high molecular weight microparticles (D4) having drug loading is provided for comparison purposes against the microparticle combination batches described in the following examples.

Figure 4 shows the release profile of D4 microparticles, exemplifying microparticles made of high molecular weight polymers. This batch was made up D4 microparticles with an 80% loading level of lidocaine. The release profile here is different from Figure 2. In this release, there are two peaks, one at 12 hours and the other at roughly 5 days. While each peak provides adequate lidocaine release, the time period between points 7 and 13, corresponding to 1.25 and 4 days respectively, provides less than 2% release. This low level is not generally adequate to relieve pain. Because high molecular weight polymers tend to release drug at a later time, it is presumed that the initial release is due to drugs on the surface of the microparticles and the later release is due to drugs coming out from the microparticles.

Comparing Figures 2 and 4, it is apparent that low and high molecular weight polymers with drug loading level produce either early or late release of drugs, causing corresponding lapse of pain relief at later or earlier time periods, respectively.

### EXAMPLE 7: PREPARATION OF D4 AND D5 POLY(DL-LACTIC-CO-GLYCOLIC ACID) (DL-PLG) MICROPARTICLE BATCHES LOADED WITH LIDOCAINE AND MIXED WITH FREE LIDOCAINE

A microparticle batch was prepared with D4 polymer, weighed at 0.5257 g, and lidocaine powder, weighed at 1.2018 g. The batch was dissolved in 2 ml of methylene chloride to create a D4/lidocaine solution. Two separate polyvinyl alcohol (PVA) solutions in water were prepared using either: (1) 0.8031 g of 98-99% hydrolyzed PVA, dissolved in 100 ml distilled water or (2) 0.2414 g of PVA, dissolved in 10 ml distilled water. An emulsion was prepared by mixing the D4/lidocaine solution and (2) PVA solution and shaking the mixture vigorously by hand in a glass vial. The resulting emulsion was transferred into a syringe with a needle. The emulsion was then introduced into a stirred (1) PVA solution. Stirring was provided by a 6 cm x 1 cm magnetic stirrer adjusted to 500 rpm. Stirring was continued for 1 hour to allow complete evaporation of the methylene chloride. Good, well formed, small (about 50 micron) microparticles were seen when observed by optical microscope. There was no crystalline lidocaine detected on the microscope slide. Stirring was stopped after about 2 hours and suspended particles were allowed to sediment undisturbed at room temperature. The clear supernatant was decanted, and microparticles collected by centrifuging followed by washing using distilled water. Even with careful drying in air with constant agitation, a significant portion of the microparticles fused (merged). The small proportion of samples that remained as microparticles during drying were used and had a theoretical drug loading level of about 70%. The release profile for the D4 microparticles is demonstrated in Figure 4. There are two peaks of release, one at 12 hours and the other at 5 days, with the release level in between mostly below 2%.

A different microparticle batch was similarly prepared using the procedure above with D5 polymer. The release profile for the D5 microparticles is demonstrated in Figure 5. This polymer is of a slightly higher molecular weight than D4. It reaches a peak release at 6 hours, most likely due to surface lidocaine, followed by a drop to 2% at 1.25 days. Then there is a sharp rise to 8% at day 2 and the release percentage stays above the 2% minimum until 5.5 days.

A microparticle combination batch was prepared using a mixture of 1.5 g of D4 microparticles, 1.5 g of D5 microparticles, and 100 mg of lidocaine free base. Lidocaine powder was reduced in particle size by grinding the powder in a mortar and pestle. This mixture was suspended in 10 ml of 2% carboxymethyl cellulose sodium with the help of vortexing (Vortex Genie; Fisher Scientific) at mark 6 for 1 minute, which became the suspension that was injected. After suspending the mixture, the blend was then divided into two equal parts of 5 ml each and placed in two 10 ml syringes.

### EXAMPLE 8: IN VITRO DRUG RELEASE FROM D1/D3/D4/D5 MICROPARTICLE COMBINATION

Table 1 shows one example of a microparticle combination. Four batches of microparticles (D1, D3, D4, D5) are shown, each with different levels of anesthetic loading, different particle size ranges, and making up a different percentage of the total combination of microparticles. For example, the D5 microparticle has the highest drug loading percentage of all four classes, the smallest particle size, and makes up the second largest percentage of microparticles in the whole combination.

**Table 1. Example of a microparticle combination using lidocaine anesthetic as the drug.**

| | Molecular Weight (MW) | Drug Loading (%)w/w | Particle Size (µm) | Amt. in Cocktail (%)w/w |
|---|---|---|---|---|
| D5 | 122,000 | 80 | 20-50 | 30 |
| D4 | 81,600 | 75 | 70-100 | 40 |
| D3 | 52,400 | 50 | 100-120 | 20 |
| D1 | 7,400 | 30 | >120 | 10 |

The formulation in Table 1 comprises in combination about 67% lidocaine.

Figure 6 shows the *in vitro* release profile of the microparticle combination shown in Table 1. A continuous level of lidocaine release can be seen from time period 1 to 20. There are three peaks in the release at time points 5, 9, and 14, which correspond to 12 hours, 2 days, and 4 days, respectively.

The release at 12 hours was the highest overall, with about 12% of the drug released at that time. This level of release provided a therapeutic effect beyond the 4-6 hours normally obtained from an injection in solution. It is believed that this release was due to drugs released from the superficial areas of the microparticles and from surface-absorbed drugs.

The release at 2 days was just over 5%. This peak represents an increased concentration of drug at the nerve surface that is necessary to maintain sodium channel blockade. This amount rejuvenated the sagging levels after 12 hours, which occurred due to drug depletion from the surface and superficial areas of microparticles, with an increase of drug release from larger particles made of lower molecular weight polymers. The structure and the increased porosity of the lower molecular weight polymers allowed for ingression of liquid which, in combination with polymer chain hydrolysis, created an increased level of drug release.

The release at 4 days was just over 7%. Polymer chain hydrolysis coupled with increased hydrolysis accounted for this observed increase in drug release. This release came mainly from the smaller microparticles made from higher molecular weight polymer. This phenomenon provided a second rejuvenation of sagging drug levels after the 2 day peak.

Between the three bursts in drug release, there was continuous release of lidocaine, with the drug levels never dropping below 3%. There was therefore continued sensory blockade beyond five days, a clear benefit not yet provide by any other invention in this area

### EXAMPLE 9: IN VITRO DRUG RELEASE FROM D4/D4 MICROPARTICLE AND FREE LIDOCAINE COMBINATION

Table 2 shows a microparticle combination with two batches of D4 microparticles and one batch of free lidocaine. Because of the range of molecular weights comprising each batch of D4 microparticles, the release profile of this combination differs between combinations, as depicted between Figures 7 and 8. However, as shown by these figures, the overall drug relief provided by these combinations extends well past 5 days.

Figure 7 shows the *in vitro* release profile of one microparticle combination depicted in Table 2. This combination was made up of 200 mg of pure lidocaine and 1.5 g. each of two batches of D4 microparticles loaded with 80% lidocaine. Slight differences exist between the two batches of D4 microparticles. As shown, there is an initial burst release of lidocaine produced by the pure lidocaine, which is followed by a steady decline over a 4-5 day period, after which an upward swing is resumed.

Figure 8 shows the *in vitro* release profile of lidocaine stemming from another microparticle combination depicted in Table 2. This microparticle combination contains 6% pure lidocaine, 47% D4 microparticles with 78.9% loading and 47% D4 microparticles with 80% loading. In this profile, there is continuous release of the drug all the way to time point 19, corresponding to 7 days. The majority of drug release does not fall below 4%, except near time point 14, corresponding to 4 days. In fact, the release does not drop below 2% until day 7, which indicates that sensory response should be prevented to this point without partial recovery to allow complete pain relief.

**Table 2. Example of another microparticle combination using lidocaine as the drug.**

| | Molecular Weight (MW) | Drug Loading (%) | Particle Size (µm) | Amt. in Cocktail (g) | Wt. % |
|---|---|---|---|---|---|
| D4-3 | 81,600 | 80 | 70-100 | 1.5 | 46.875 |
| D4-7 | 81,600 | 80 | 70-100 | 1.5 | 46.875 |
| Lidocaine Free base, drug | | 100 | 50-100 | 200 (mg) | 6.25 |

### EXAMPLE 10: IN VITRO DRUG RELEASE FROM D5/D3/D1/D1/D1 MICROPARTICLE COMBINATION

Figure 9 shows the *in vitro* release profile of a microparticle combination depicted in Table 3. This combination is made up of 1.2 g of D1 (batch 033006), 600 mg of a second batch of D1 (batch 022406), 600 mg of D3 (batch 041906), and 600 mg of D5 (batch 030306). The percent loading of lidocaine for each group of microparticles is shown in the table. As shown in the figure, there is an initial higher burst release of lidocaine produced by lidocaine on the surface of all 5 batches of microparticles. This release is followed by a rapid decline over a 6 day period and then a short upward swing due to the D4 microparticle. Overall, the percent lidocaine released does not fall below 2% until day 8.

**Table 3. Microparticle combination of 4 batches having lidocaine**

| | Molecular Weight (MW) | Drug Loading (%)w/w | Particle Size (µm) | Amt. in Cocktail (%)w/w |
|---|---|---|---|---|
| D5 | 122,000 | 57.8 | 20-50 | 20 |
| D3 | 52,400 | 80 | 70-100 | 20 |
| D1 (033006) | 7,400 | 70 | 100-120 | 40 |
| D1 (022406) | 7,400 | 70 | 100-120 | 20 |

### EXAMPLE 11: IN VIVO DRUG RELEASE FROM D4/D4 MICROPARTICLE AND FREE LIDOCAINE COMBINATION

The microparticle combination in Example 9 and depicted in Figure 7 was also injected in an *in vivo* study in sheep.

The *in vivo* study showed a detectable serum lidocaine level of 1 mcg/ml in the sample taken 2 hours after injection, which is sufficient to cause motor blockade. Subsequent samples taken produced less than 0.5 mcg/ml of lidocaine. However, the drug concentration in tissue surrounding the injection site was high enough to cause recoverable sensory blockade after motor blockade ended 2-4 hours after injection.

Both the *in vitro* and *in vivo* studies using the microparticle combination in Table 2 therefore show corroborative data. Results from the *in vivo* study (data not presented) show a partial recovery of the sensory response in sheep on day 5 (corresponding to the end of the 4-5 day decline *in vitro*), followed by an immediate re-establishment of the sensory block lasting for an additional 3.5 days (corresponding to the upward swing results in the *in vitro data*). The microparticle combination was still releasing about 2% of 2.6 g of lidocaine *in vivo* after 7.5 days, which is similar to that released after the initial 0.5 hour following injection. This amount appears to be the approximate amount necessary to be injected for continuous release in sheep in order to maintain sensory response suppression.

### EXAMPLE 12: ELECTRON MICROSCOPE PICTURES OF DIFFERENT MICROPARTICLES LOADED WITH LIDOCAINE

Figures 10-13 illustrate electron microscope pictures of, D1, D2, D3, and D4 microparticles respectively. Each of the microparticles were loaded with 80% lidocaine, according to the procedures described above. D1 and D2 microparticles, which have lower molecular weight polymers, did not form discreet injectable microparticles as did D3 and D4.

## Claims

1. A composition comprising:
a plurality of microparticles,
wherein substantially all of the plurality of microparticles comprise one or more local anesthetic compounds,
wherein at least some of the plurality of microparticles comprise at least one polymer from which the local anesthetic compound is releasable,
wherein at least some of the plurality of microparticles comprise one or more local anesthetic compounds in an amount of at least 70 % by weight,
wherein the average amount of local anesthetic compound in the composition is at least 50 % by weight, and
wherein the composition optionally further comprises a local anesthetic effect-augmentation agent which, if present, is included in the composition in an amount of less than 0.005 % by weight,
**characterized in that** the plurality of microparticles comprises a mixture of at least two groups of microparticles, each group having an average polymer molecular weight, an average drug loading percentage, and an average particle size, wherein at least the average polymer molecular weight is different in each of the groups, wherein at least one of the local anesthetic compounds is chosen from the group consisting of: lidocaine, bupivacaine, ropivacaine, dibucaine, etidocaine, tetracaine, xylocaine, procaine, chloroprocaine, prilocaine, mepivacaine, mixtures thereof and salts thereof, and wherein the at least one polymer is chosen from the group consisting of polyesters, polyorthoesters, proteins, polysaccharides, and combinations thereof, poly(lactic) acid, poly(glycolic) acid, polyactide,polyglycolides, poly(DL-lactic-co-glycolic) acid, polyanhydride, polycaprolactone, and polyphosphazene.

2. A composition according to claim 1 comprising:
(a) a first group of microparticles, each microparticle in said first group having a molecular weight greater than 91,600 Daltons, a particle size between 20 and 50 microns, and a drug loading of at least one anesthetic of 80 % by weight;
(b) a second group of microparticles, each microparticle in said second group having a molecular weight between 57,600 and 91,600 Daltons, a particle size between 70 and 100 microns, and a drug loading of said at least one anesthetic of 75 % by weight;
(c) a third group of microparticles, each microparticle in said third group having a molecular weight between 31,300 and 57,600 Daltons, a particle size between 100 and 120 microns, and a drug loading of said at least one anesthetic of 50 % by weight; and
(d) a fourth group of microparticles, each microparticle in said fourth group having a molecular weight between 5,000 and 12,900 Daltons, a particle size greater than 120 microns, and a drug loading of said at least one anesthetic of 30 % by weight,
wherein said first group comprises 30 %, said second group comprises 40 %, said third group comprises 20 %, and said fourth group comprises 10 % of the total microparticles of all four groups.

3. A composition according to claim 1 comprising:
(a) a first group of microparticles, each microparticle in said first group having a molecular weight between 57,600 and 91,600 Daltons, a particle size between 70 and 100 microns, and a drug loading of at least one anesthetic of at least 80 % by weight;
(b) a second group of microparticles, each microparticle in said second group having a molecular weight between 57,600 and 91,600 Daltons, a particle size between 70 and 100 microns, and a drug loading of said at least one anesthetic of at least 80 % by weight; and
(c) said at least one anesthetic in free form, each anesthetic particle in free form having a particle size between 50 and 100 microns,
wherein said first group comprises 47 %, said second group comprises 47 %, and said third group comprises 6 % of the total mass of elements (a), (b), and (c).

4. A composition according to claim 1 comprising:
(a) a first group of microparticles, each microparticle in said first group having a molecular weight greater than 91,600 Daltons, a particle size between 20 and 50 microns, and a drug loading of at least one anesthetic of 58 % by weight;
(b) a second group of microparticles, each microparticle in said second group having a molecular weight between 57,600 and 91,600 Daltons, a particle size between 70 and 100 microns, and a drug loading of said at least one anesthetic of 80 % by weight;
(c) a third group of microparticles, each microparticle in said third group having a molecular weight between 5,000 and 12,900 Daltons, a particle size between 100 and 120 microns, and a drug loading of said at least one anesthetic of 70 % by weight; and
(d) a fourth group of microparticles, being different from said third group of microparticles, each microparticle in said fourth group also having a molecular weight between 5,000 and 12,900 Daltons, a particle size between 100 and 120 microns, and a drug loading of said at least one anesthetic of 70 % by weight,
wherein said first group comprises 20 %, said second group comprises 20 %, said third group comprises 40 %, and said fourth group comprises 20 % of the total microparticles of all four groups.

5. The composition according to claim 1, wherein the composition is totally free of local anesthetic effect-augmentation agent.

6. The composition according to claim 1, wherein at least some of the plurality of microparticles comprise the local anesthetic compound substantially free of the polymer.

7. The composition according to claim 1, wherein substantially all of the microparticles that comprise polymer also comprise at least 70 % by weight of the local anesthetic compound.

8. The composition according to claim 1, wherein at least 90 % by weight of the microparticles comprise at least 60 % by weight of the local anesthetic compound.

9. The composition according to claim 1, wherein the at least one polymer is poly(DL-lactic-co-glycolic) acid.

10. The composition according to claim 11, wherein at least one of the local anesthetic compounds is lidocaine.

11. The composition according to claim 1, the at least one polymer is poly(DL-lactic-co-glycolic) acid, and at least one of the local anaesthetic compounds is lidocaine.

12. A method of making drug-loaded microparticles, comprising:
(a) providing at least one anesthetic chosen from the group consisting of: lidocaine, bupivacaine, ropivacaine, dibucaine, etidocaine, tetracaine, xylocaine, procaine, chloroprocaine, prilocaine, mepivacaine, mixtures thereof and salts thereof;
(b) providing at least one polymer chosen from the group consisting of polyesters, polyorthoesters, proteins, polysaccharides, and combinations thereof, poly(lactic) acid, poly(glycolic) acid, polyactide, polyglycolide, poly(DL-lactic-co-glycolic) acid, polyanhydride, polycaprolactone, and polyphosphazene;
(c) dissolving said at least one anesthetic and said at least one polymer in an organic solvent to produce a solution;
(d) emulsifying said solution by stirring it into an aqueous medium to form an oil-in-water emulsion;
(e) evaporating said organic solvent to allow said at least one anesthetic and said at least one polymer to harden into a batch of microparticles;
(f) repeating steps (a) through (e) to produce multiple batches of microparticles,
wherein each batch comprises microparticles within a distinct size range, wherein each batch makes up a different percentage of the combination of all of the batches, and
wherein each batch comprises said at least one anesthetic at a different loading level.

## Patentansprüche

1. Zusammensetzung umfassend:
eine Mehrzahl von Mikropartikeln,
wobei im Wesentlichen alle der Mehrzahl von Mikropartikeln eine oder mehrere Lokalanästhetikverbindungen umfassen,
wobei mindestens einige der Mehrzahl von Mikropartikeln mindestens ein Polymer umfassen, aus dem die Lokalanästhetikverbindung freisetzbar ist,
wobei mindestens einige der Mehrzahl von Mikropartikeln eine oder mehrere Lokalanästhetikverbindungen in einer Menge von mindestens 70 Gew.-% umfassen,
wobei die durchschnittliche Menge an Lokalanästhetikverbindung in der Zusammensetzung mindestens 50 Gew.-% beträgt und
wobei die Zusammensetzung wahlweise ferner ein die Lokalanästhetikwirkung erhöhendes Mittel umfasst, das, liegt es vor, in die Zusammensetzung in einer Menge von weniger als 0,005 Gew.-% integriert ist,
**dadurch gekennzeichnet, dass** die Mehrzahl von Mikropartikeln eine Mischung von mindestens zwei Gruppen von Mikropartikeln umfasst, wobei jede Gruppe ein durchschnittliches Polymermolekulargewicht, einen durchschnittlichen Arzneimittelbeladungsprozentsatz und eine durchschnittliche Partikelgröße aufweist, wobei mindestens das durchschnittliche Polymermolekulargewicht in jeder der Gruppen verschieden ist, wobei mindestens eine der Lokalanästhetikverbindungen aus der Gruppe ausgewählt wird bestehend aus: Lidocain, Bupivacain, Ropivacain, Dibucain, Etidocain, Tetracain, Xylocain, Procain, Chloroprocain, Prilocain, Mepivacain, Mischungen davon und Salzen davon und wobei das mindestens eine Polymer aus der Gruppe ausgewählt wird bestehend aus Polyestern, Polyorthoestern, Proteinen, Polysacchariden und Kombinationen davon, Poly(milch)säure, Poly(glycol)säure, Polylactid, Polyglycoliden, Poly(DL-milch-co-glycol)säure, Polyanhydrid, Polycaprolacton und Polyphosphazen.

2. Zusammensetzung nach Anspruch 1, umfassend:
(a) eine erste Gruppe von Mikropartikeln, wobei jedes Mikropartikel in der ersten Gruppe ein Molekulargewicht über 91.600 Dalton, eine Partikelgröße zwischen 20 und 50 Mikron und eine Arzneimittelbeladung von mindestens einem Anästhetikum von 80 Gew.-% aufweist;
(b) eine zweite Gruppe von Mikropartikeln, wobei jedes Mikropartikel in der zweiten Gruppe ein Molekulargewicht zwischen 57.600 und 91.600 Dalton, eine Partikelgröße zwischen 70 und 100 Mikron und eine Arzneimittelbeladung des mindestens einen Anästhetikums von 75 Gew.-% aufweist;
(c) eine dritte Gruppe von Mikropartikeln, wobei jedes Mikropartikel in der dritten Gruppe ein Molekulargewicht zwischen 31.300 und 57.600 Dalton, eine Partikelgröße zwischen 100 und 120 Mikron und eine Arzneimittelbeladung des mindestens einen Anästhetikums von 50 Gew.-% aufweist; und
(d) eine vierte Gruppe von Mikropartikeln, wobei jedes Mikropartikel in der vierten Gruppe ein Molekulargewicht zwischen 5.000 und 12.900 Dalton, eine Partikelgröße über 120 Mikron und eine Arzneimittelbeladung des mindestens einen Anästhetikums von 30 Gew.-% aufweist;
wobei die erste Gruppe 30 %, die zweite Gruppe 40 %, die dritte Gruppe 20 % und die vierte Gruppe 10 % der gesamten Mikropartikel aller vier Gruppen umfasst.

3. Zusammensetzung nach Anspruch 1, umfassend:
(a) eine erste Gruppe von Mikropartikeln, wobei jedes Mikropartikel in der ersten Gruppe ein Molekulargewicht zwischen 57.600 und 91.600 Dalton, eine Partikelgröße zwischen 70 und 100 Mikron und eine Arzneimittelbeladung von mindestens einem Anästhetikum von 80 Gew.-% aufweist;
(b) eine zweite Gruppe von Mikropartikeln, wobei jedes Mikropartikel in der zweiten Gruppe ein Molekulargewicht zwischen 57.600 und 91.600 Dalton, eine Partikelgröße zwischen 70 und 100 Mikron und eine Arzneimittelbeladung des mindestens einen Anästhetikums von mindestens 80 Gew.-% aufweist; und
(c) das mindestens eine Anästhetikum in freier Form, wobei jedes Anästhetikumpartikel in freier Form eine Partikelgröße zwischen 50 und 100 Mikron aufweist,
wobei die erste Gruppe 47 %, die zweite Gruppe 47 % und die dritte Gruppe 6 % der Gesamtmasse der Elemente (a), (b) und (c) umfasst.

4. Zusammensetzung nach Anspruch 1, umfassend:
(a) eine erste Gruppe von Mikropartikeln, wobei jedes Mikropartikel in der ersten Gruppe ein Molekulargewicht über 91.600 Dalton, eine Partikelgröße zwischen 20 und 50 Mikron und eine Arzneimittelbeladung von mindestens einem Anästhetikum von 58 Gew.-% aufweist;
(b) eine zweite Gruppe von Mikropartikeln, wobei jedes Mikropartikel in der zweiten Gruppe ein Molekulargewicht zwischen 57.600 und 91.600 Dalton, eine Partikelgröße zwischen 70 und 100 Mikron und eine Arzneimittelbeladung des mindestens einen Anästhetikums von 80 Gew.-% aufweist;
(c) eine dritte Gruppe von Mikropartikeln, wobei jedes Mikropartikel in der dritten Gruppe ein Molekulargewicht zwischen 5.000 und 12.900 Dalton, eine Partikelgröße zwischen 100 und 120 Mikron und eine Arzneimittelbeladung des mindestens einen Anästhetikums von 70 Gew.-% aufweist; und
(d) eine vierte Gruppe von Mikropartikeln, die von der dritten Gruppe von Mikropartikeln verschieden ist, wobei jedes Mikropartikel in der vierten Gruppe ebenfalls ein Molekulargewicht zwischen 5.000 und 12.900 Dalton, eine Partikelgröße zwischen 100 und 120 Mikron und eine Arzneimittelbeladung des mindestens einen Anästhetikums von 70 Gew.-% aufweist;
wobei die erste Gruppe 20 %, die zweite Gruppe 20 %, die dritte Gruppe 40 % und die vierte Gruppe 20 % der gesamten Mikropartikel aller vier Gruppen umfasst.

5. Zusammensetzung nach Anspruch 1, wobei die Zusammensetzung vollkommen frei von Lokalanästhetikwirkung erhöhendem Mittel ist.

6. Zusammensetzung nach Anspruch 1, wobei mindestens einige der Mehrzahl von Mikropartikeln die Lokalanästhetikverbindung, die im Wesentlichen frei von dem Polymer ist, umfasst.

7. Zusammensetzung nach Anspruch 1, wobei im Wesentlichen alle der Mikropartikel, die Polymer umfassen, auch mindestens 70 Gew.-% Lokalanästhetikverbindung umfassen.

8. Verbindung nach Anspruch 1, wobei mindestens 90 Gew.-% der Mikropartikel mindestens 60 Gew.-% der Lokalanästhetikverbindung umfassen.

9. Zusammensetzung nach Anspruch 1, wobei das mindestens eine Polymer Poly(DL-milch-co-glycol)säure ist.

10. Zusammensetzung nach Anspruch 11, wobei mindestens eine der Lokalanästhetikverbindungen Lidocain ist.

11. Zusammensetzung nach Anspruch 1, wobei mindestens ein Polymer Poly(DL-milch-co-glycol)säure ist und mindestens eine der Lokalanästhetikverbindungen Lidocain ist.

12. Verfahren zum Herstellen von mit Arzneimittel beladenen Mikropartikeln, umfassend:
(a) Bereitstellen mindestens eines Anästhetikums ausgewählt aus der Gruppe bestehend aus Lidocain, Bupivacain, Ropivacain, Dibucain, Etidocain, Tetracain, Xylocain, Procain, Chloroprocain, Prilocain, Mepivacain, Mischungen davon und Salzen davon;
(b) Bereitstellen mindestens eines Polymers ausgewählt aus der Gruppe bestehend aus Polyestern, Polyorthoestern, Proteinen, Polysacchariden und Kombinationen davon, Poly(milch)säure, Poly(glycol)säure, Polylactid, Polyglycoliden, Poly(DL-milch-co-glycol)säure, Polyanhydrid, Polycaprolacton und Polyphosphazen;
(c) Lösen des mindestens einen Anästhetikums und des mindestens einen Polymers in einem organischen Lösungsmittel, um eine Lösung herzustellen;
(d) Emulgieren der Lösung durch Rühren derselben in ein wässriges Medium, um eine Öl-in-Wasser-Emulsion zu bilden;
(e) Verdampfen des organischen Lösungsmittels, um zu gestatten, dass das mindestens eine Anästhetikum und das mindestens eine Polymer sich zu einer Charge Mikropartikel härten;
(f) Wiederholen der Schritte (a) bis (e), um multiple Chargen von Mikropartikeln herzustellen,
wobei jede Charge Mikropartikel innerhalb eines spezifischen Größenbereichs umfasst,
wobei jede Charge einen anderen Prozentsatz der Kombination aller der Umweg Chargen ausmacht und
wobei jede Charge das mindestens eine Anästhetikum in einem anderen Beladungsniveau umfasst.

## Revendications

1. Composition comprenant:
une pluralité de microparticules,
dans laquelle substantiellement la totalité de la pluralité de microparticules comprend un ou plusieurs composés anesthésiques locaux,
dans laquelle au moins une partie de la pluralité de microparticules comprend au moins un polymère à partir duquel le composé anesthésique local peut être libéré,
dans laquelle au moins une partie de la pluralité de microparticules comprend un ou plusieurs composés anesthésiques locaux dans une quantité d'au moins 70% en poids,
où la quantité moyenne de composé anesthésique local dans la composition est d'au moins 50% en poids, et
où la composition comprend en outre optionnellement un agent d'augmentation d'effet anesthésique local qui, s'il est présent, est inclus dans la composition dans une quantité de moins de 0,005% en poids,
**caractérisée en ce que** la pluralité de microparticules comprend un mélange d'au moins deux groupes de microparticules, chaque groupe possédant un poids moléculaire de polymère moyen, un pourcentage de chargement de médicament moyen et une taille de particule moyenne, dans laquelle au moins le poids moléculaire de polymère moyen est différent dans chacun des groupes, dans laquelle au moins un des composés anesthésiques locaux est choisi dans le groupe constitué de: lidocaïne, bupivacaïne, ropivacaïne, dibucaïne, étidocaïne, tétracaïne, xylocaïne, procaïne, chloroprocaïne, prilocaïne, mépivacaïne, leurs mélanges et leurs sels et dans laquelle le au moins un polymère est choisi dans le groupe constitué de: polyesters, polyorthoesters, protéines, polysaccharides et leurs combinaisons, acide poly(lactique), acide poly(glycolique), polylactide, polyglycolides, acide poly(DL-lactique-co-glycolique), polyanhydride, polycaprolactone et polyphosphazène.

2. Composition selon la revendication 1, comprenant:
(a) un premier groupe de microparticules, chaque microparticule dans ledit premier groupe possédant un poids moléculaire supérieur à 91.600 Daltons, une taille de particule entre 20 et 50 microns et un chargement de médicament d'au moins un anesthésique de 80% en poids;
(b) un deuxième groupe de microparticules, chaque microparticule dans ledit deuxième groupe possédant un poids moléculaire entre 57.600 et 91.600 Daltons, une taille de particule entre 70 et 100 microns et un chargement de médicament dudit au moins un anesthésique de 75% en poids;
(c) un troisième groupe de microparticules, chaque microparticule dans ledit troisième groupe possédant un poids moléculaire entre 31.300 et 57.600 Daltons, une taille de particule entre 100 et 120 microns et un chargement de médicament dudit au moins un anesthésique de 50% en poids; et
(d) un quatrième groupe de microparticules, chaque microparticule dans ledit quatrième groupe possédant un poids moléculaire entre 5.000 et 12.900 Daltons, une taille de particule supérieure à 120 microns et un chargement de médicament dudit au moins un anesthésique de 30% en poids,
dans laquelle ledit premier groupe comprend 30%, ledit deuxième groupe comprend 40%, ledit troisième groupe comprend 20% et ledit quatrième groupe comprend 10% des microparticules totales de la totalité des quatre groupes.

3. Composition selon la revendication 1, comprenant:
(a) un premier groupe de microparticules, chaque microparticule dans ledit premier groupe possédant un poids moléculaire entre 57.600 et 91.600 Daltons, une taille de particule entre 70 et 100 microns et un chargement de médicament d'au moins un anesthésique d'au moins 80% en poids;
(b) un deuxième groupe de microparticules, chaque microparticule dans ledit deuxième groupe possédant un poids moléculaire entre 57.600 et 91.600 Daltons, une taille de particule entre 70 et 100 microns et un chargement de médicament dudit au moins un anesthésique d'au moins 80% en poids; et
(c) ledit au moins un anesthésique sous une forme libre, chaque particule d'anesthésique sous une forme libre possédant une taille de particule entre 50 et 100 microns,
dans laquelle ledit premier groupe comprend 47%, ledit deuxième groupe comprend 47% et ledit troisième groupe comprend 6% de la masse totale des éléments (a), (b) et (c).

4. Composition selon la revendication 1, comprenant:
(a) un premier groupe de microparticules, chaque microparticule dans ledit premier groupe possédant un poids moléculaire supérieur à 91.600 Daltons, une taille de particule entre 20 et 50 microns et un chargement de médicament d'au moins un anesthésique de 58% en poids;
(b) un deuxième groupe de microparticules, chaque microparticule dans ledit deuxième groupe possédant un poids moléculaire entre 57.600 et 91.600 Daltons, une taille de particule entre 70 et 100 microns et un chargement de médicament dudit au moins un anesthésique de 80% en poids;
(c) un troisième groupe de microparticules, chaque microparticule dans ledit troisième groupe possédant un poids moléculaire entre 5.000 et 12.900 Daltons, une taille de particule entre 100 et 120 microns et un chargement de médicament dudit au moins un anesthésique de 70% en poids; et
(d) un quatrième groupe de microparticules, qui est différent dudit troisième groupe de microparticules, chaque microparticule dans ledit quatrième groupe possédant également un poids moléculaire entre 5.000 et 12.900 Daltons, une taille de particule entre 100 et 120 microns et un chargement de médicament dudit au moins un anesthésique de 70% en poids,
dans laquelle ledit premier groupe comprend 20%, ledit deuxième groupe comprend 20%, ledit troisième groupe comprend 40% et ledit quatrième groupe comprend 20% des microparticules totales de la totalité des quatre groupes.

5. Composition selon la revendication 1, où la composition est totalement exempte d'agent d'augmentation d'effet anesthésique local.

6. Composition selon la revendication 1, dans laquelle au moins une partie de la pluralité de microparticules comprend le composé anesthésique local substantiellement exempt du polymère.

7. Composition selon la revendication 1, dans laquelle substantiellement la totalité des microparticules qui comprennent un polymère comprend également au moins 70% en poids du composé anesthésique local.

8. Composition selon la revendication 1, dans laquelle au moins 90% en poids des microparticules comprennent au moins 60% en poids du composé anesthésique local.

9. Composition selon la revendication 1, dans laquelle le au moins un polymère est l'acide poly(DL-lactique-co-glycolique).

10. Composition selon la revendication 11, dans laquelle au moins un des composés anesthésiques locaux est la lidocaïne.

11. Composition selon la revendication 1, dans laquelle le au moins un polymère est l'acide poly(DL-lactique-co-glycolique) et au moins un des composés anesthésiques locaux est la lidocaïne.

12. Méthode pour la fabrication de microparticules chargées de médicament, comprenant:
(a) la fourniture d'au moins un anesthésique choisi dans le groupe constitué de: lidocaïne, bupivacaïne, ropivacaïne, dibucaïne, étidocaïne, tétracaïne, xylocaïne, procaïne, chloroprocaïne, prilocaïne, mépivacaïne, leurs mélanges et leurs sels;
(b) la fourniture d'au moins un polymère choisi dans le groupe constitué de: polyesters, polyorthoesters, protéines, polysaccharides et leurs combinaisons, acide poly(lactique), acide poly(glycolique), polylactide, polyglycolide, acide poly(DL-lactique-co-glycolique), polyanhydride, polycaprolactone et polyphosphazène;
(c) la dissolution dudit au moins un anesthésique et dudit au moins un polymère dans un solvant organique pour produire une solution;
(d) l'émulsification de ladite solution en l'agitant dans un milieu aqueux pour former une émulsion d'huile dans l'eau;
(e) l'évaporation dudit solvant organique pour permettre audit au moins un anesthésique et audit au moins un polymère de durcir en un lot de microparticules;
(f) la répétition des étapes (a) à (e) pour produire de multiples lots de microparticules,
dans laquelle chaque lot comprend des microparticules dans une gamme de taille distincte,
dans laquelle chaque lot constitue un pourcentage différent de la combinaison de la totalité des lots, et
dans laquelle chaque lot comprend ledit au moins un anesthésique à un niveau de chargement différent.
